# EUROPEAN PATENT APPLICATION

(11) **EP 2 649 992 A1**
(43) Date of publication of application: **16.10.2013**
(21) Application number: 10860501.5
(22) Date of filing: 08.12.2010
(51) Int. Cl.: A61K 31/167, A61P 37/06

(54) **NEW PHARMACEUTIC USE OF BENZOIC AICD DERIVATIVES**

(71) Applicant: Li, Xianliang, Beijing 102206 (CN); Li, Yingji, Beijing 102206 (CN)
(72) Inventor: LI, Xianliang, Changping Beijing 102206 (CN)
(74) Representative: Kalhammer, Georg
(86) International application number: PCT/CN2010/001994
(87) International publication number: WO 2012/075606

(57) **Abstract**

The present invention relates to a novel pharmaceutical use of benzoic acid derivatives, further to a novel pharmaceutical use of cinnamon anthranilic acid type drugs, specifically to a use of cinnamon anthranilic acid and its derivatives, or a salt thereof, or a composition comprising the above in the preparation of a drug for anti-acute rejection or for the induction of immune tolerance.

## Description

### TECHNICAL FIELD

The present invention relates to a novel pharmaceutical use of benzoic acid derivatives, further to a novel pharmaceutical use of cinnamon anthranilic acid type drugs, specifically to a use of cinnamon anthranilic acid and its derivatives, or a salt thereof, or a composition comprising the above in the preparation of a drug for anti-acute rejection or for the induction of immune tolerance.

### TECHNICAL BACKGROUND

Transplantation, especially organ transplantation after the development of nearly 60 years, with the development of immunosuppressive drugs and organ transplantation technology, has been able to successfully control most of the acute rejections, and become a routine treatment for some clinical end-stage diseases. However, regardless how hard people work on it, long term survival of recipients remains difficult to be improved by using the existing clinical immunosuppressive drugs. Due to the immune rejection factors or toxic side effects of immunosuppressive drugs, 5-15% of transplanted organs loss the function every year, and became the bottleneck of the long-term survival of transplant patients and grafts. Current immunosuppressive drugs, especially calcineurin inhibitors (CNI) such as cyclosporin A and FK506, etc., not only have severe kidney toxicity and other serious side effects, but also suppress the generation as well as division and proliferation of regulatory cells. In this sense, calcineurin inhibitors may become an obstacle to achieve long-term survival of grafts and the induction of immune tolerance. Rapamycin, compared with calcineurin inhibitor, can enhance the proliferation activity and function of regulatory T cells, but there is no sign of correlation between increased naive regulatory T cells and function maintenance of a graft. Therefore, seeking drugs and treatment programs which are able to induce the generation of donor-specific regulatory cells and to induce immune tolerance mediated by such cells is the key to ultimately solve this problem and has become a research focus in the field of transplantation.

Immune tolerance in transplantation is defined as : without of taking any immunosuppressive agent and maintaining a patient's normal immune functions to achieve a state, that a recipient has no destructive immune response to a graft, so as to maintain the function of the graft stable. The presence of the state of immune tolerance in organ transplantation provides a strategy of obtaining immune tolerance and also a new direction for the treatment of autoimmune diseases and allergic diseases. Conditions of many autoimmune diseases are alleviated through immune reconstitution or immune tolerance therapy.

Currently, clinical trials on active induction of immune tolerance in organ transplant have already started in some clinical research centers. Collaborative research at the international immune tolerance cooperation and coordination multi-center shows that about 20% of liver transplant selected cases can successfully stop using immunosuppressive drugs, and maintain good function of a graft. It means that active induction of immune tolerance in transplantation is not only feasible, but also has a promising future.

However, the success of above mentioned immune tolerance cannot be well explained by immune chimeric theory. Thus, currently active induction of immune tolerance in patients has certain blindness in theory clinically. Because the treatment program of induction of immune chimerism does not bring our expected immune tolerance, and in a recipient with immune tolerance, the amount of immune chimeric cells is not associated with the function of a transplanted organ.

Researches on regulatory cells in recent years prompted a new theory basis that a regulatory cell is the key to successfully achieve the induction of immune tolerance. Please see, (Garden OA, Pinheiro D, Cunningham F.All creatures great and small: Regulatory T cells in mice, humans, dogs and other domestic animal species. Int Immunopharmacol. 2010 Nov 17[Epub ahead of print]; Nafady-Hego H, Li Y, Ohe H,The generation of donor-specific CD4+CD25++CD45RA+ naive regulatory T Cells in operationally tolerant patients after pediatric living-donor liver transplantation. Transplantation. 2010 Nov 16. [Epub ahead of print])

Regulatory cells are a class of cell populations with relatively low proliferation ability and able to suppress immune response. They play an important role in immune pathology, graft tolerance, autoimmune reaction prevention and organism immune balance maintenance. Common regulatory cells include CD4+CD25+regulatory T cells, CD8+regulatory T cells, regulatory B cells, regulatory NK cells, and regulatory dendritic cells, etc. Regulatory cells also can adoptively transfer the state of immune tolerance in vivo from one recipient to another recipient, or adoptively transfer to another recipient after cell culture in vitro. Transfer of immune tolerance in vivo is generally considered as a result of cell cytotoxic killing or inhibition of cell proliferation.

Donor-specific regulatory cells can mediate immune tolerance, and adoptive transfer of immunogenic regulatory cells can induce the generation of immune tolerance, and have been well documented in many organ transplant models. In recent years, international clinical studies on patients with immune tolerance have also focused on the induction, generation, and transfer of regulatory cells in transplant patients with immune tolerance, and the generation of regulatory cells are relative with a graft's function and long-term survival of the graft.

In 2004, Kyoto Organ Transplant Centre in Japan found, in the peripheral blood of liver transplant patients with immune tolerance, the amount of CD4+CD25^{high} regulatory cells, B cells and δ1-γδTCR+T cells significantly increased. Pons found in the peripheral blood of liver transplant patients with immune tolerance, the amount of CD4+CD25^{high} regulatory cells significantly increased with significantly increased expression of Foxp³. Multicenter studies on kidney transplant patients with immune tolerance at the French Institute have shown that CD8+ regulatory cells play an important role in immune tolerance of kidney transplant. In 2008, Trzonkowski found the existence of immunosuppressive CD8+CD28-cells in kidney transplant patients. In the observation of some other clinical long-term survival of organ transplant recipients, and patients with immune tolerance who don't require the use of immunosuppressive agents, the function of CD8+ regulatory cells attracts more and more attention. There are reports regarding regulatory cells in patients with immune tolerance in kidney transplantation, heart transplantation, and liver and small bowel joint transplantation.

Within current immunosuppressive drugs for clinical applications, no drug can clearly promote the generation of donor-specific regulatory cells so as to induce immune tolerance.

Cinnamon anthranilic acid, its chemical structure is formula I:

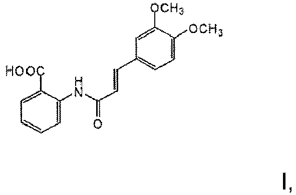

Its chemical name is 2-[[[3-(3,4-dimethoxy)phenyl-1-oxo-2-propenyl]amino]benzoic acid; and its trade name is Tranilast. It is one of degradation metabolites of tryptophan by indoleamine 2,3-dioxygenase (IDO) or tryptophan 2,3-dioxygenase (TDO), which once was widely used against allergy, such as the treatment of respiratory asthma, allergic rhinitis, atopic dermatitis, allergic conjunctivitis, etc., and against skin diseases, such as keloids and hypertrophic scars, etc. Because this type of drugs were found to inhibit chemical media causing allergic reactions, but also to inhibit the excessive collagen accumulation in skin tissues caused by fibroblasts.

Japanese patent applications Hei-6-135829 and Hei-9-227371 disclose cinnamon anthranilic acid can inhibit induced excessive proliferation of vascular smooth muscle cells after percutaneous transluminal coronary angioplasty (PTCA), and be used for the treatment and prevention of coronal vascular restenosis caused by PTCA. This type of drugs also can reduce the deposition of atherosclerosis, thus to inhibit the formation of atherosclerosis.

U.S. Patent U.S. 6552083B1 discloses cinnamon anthranilic acid type drugs can inhibit the thickening of blood vessels in a transplanted heart, and inhibit the infiltration of lymphocytes to the graft, and thus be used in anti-chronic rejection treatment of organ transplantation.

United States patent application US 2010/0158905 A1 discloses cinnamon anthranilic acid type drugs can inhibit the proliferation of lymphocytes, and thus be used for the treatment of rheumatoid arthritis.

It has not yet been found the use of cinnamon anthranilic acid type drugs in the treatment of anti-acute rejection after the transplantation and in the induction of immune tolerance.

### SUMMARY OF THE INVENTION

The inventors of this application studied cinnamon anthranilic acid type drugs in a rodent organ transplant model, and found that cinnamon anthranilic acid type drugs were able to protect most grafts to prevent acute rejection and to prolong the survival term of the graft, and cinnamon anthranilic acid type drugs also made transplant recipients to generate a status of immune tolerance. Further studies revealed that cinnamon anthranilic acid type drugs can induce the generation of regulatory cells in a transplant recipient; and studies also revealed that by adoptively transferring the regulatory cells, it can also make a transplant recipient to generate a status of immune tolerance. The present invention for the first time found that cinnamon anthranilic acid type drugs can promote the generation of donor-specific regulatory cells.

Thus, the present invention provides a novel pharmaceutical use of benzoic acid derivatives, further to a novel pharmaceutical use of cinnamon anthranilic acid type drugs, i.e. a use of cinnamon anthranilic acid and its derivatives, or a salt thereof, or a composition comprising the above in the preparation of a drug for the prevent of acute rejection or the induction of immune tolerance after transplantation.

In one embodiment, the present invention also provides the use of cinnamon anthranilic acid and its derivatives, or a salt thereof, or a composition comprising the above in the preparation of a drug for inducing a transplant recipient to generate regulatory cells.

In one embodiment, the present invention provides the use of cinnamon anthranilic acid and its derivatives, or a salt thereof, or a composition comprising the above in the preparation of a drug for post-transplantation immune tolerance, and a therapeutically effective amount of cinnamon anthranilic acid and its derivatives, or a salt thereof, or a composition comprising the above is directly administered to a recipient.

In another embodiment, the present invention provides the use of cinnamon anthranilic acid and its derivatives, or a salt thereof, or a composition comprising the above in the preparation of a drug for post transplantation immune tolerance; first, a therapeutically effective amount of cinnamon anthranilic acid and its derivatives or a salt thereof, or a composition comprising the above is used in mix cultured donor and recipient cells to induce the generation of regulatory cells, and then a therapeutically effective concentration of regulatory cells are adoptively transferred to the recipient. In this use, for example, a blood cell separator can be used to collect peripheral blood mononuclear cells from the donor and the recipient. The amount of cells shall be ensured to be at least greater than 1×10⁹ cells. Then donor and recipient cells are used in proportion for mixed lymphocyte culture, and are treated with cinnamon anthranilic acid and its derivatives, or a salt thereof or likewise suitable drugs. Meanwhile, various cytokines are applied for induction and stimulation, to ensure the induction of donor-specific lymphocyte subsets. Then based on surface labels of the regulatory cells, the regulatory cells are collected using immuno-magnetic bead negative selection method for adoptive transfer. They are directly transferred to the recipient through peripheral vascular. For example, it can reference to: (Fändrich F.Cell therapy approaches aiming at minimization of immunosuppression in solid organ transplantation. Curr Opin Organ Transplant. 2010 Oct 7. [Epub ahead of print] ; Shuiping Jiang, Julia Tsang, Paul Tam. Regulatory T cell immunotherapy for transplantation tolerance: Step into clinic. International Immunopharmacology 2010;10:1486-1490)

Further, the present invention provides the use of cinnamon anthranilic acid and its derivatives, or a salt thereof, or a composition comprising the above in the preparation of a drug for anti-acute rejection post-transplantation; the use of cinnamon anthranilic acid and its derivatives, or a salt thereof, or a composition comprising the above in the preparation of a drug for anti-acute rejection post-transplantation includes, but is not limited to, a therapeutically effective amount of cinnamon anthranilic acid and its derivatives, or a salt thereof, or a composition comprising the above is directly administered to a recipient.

According to the uses of the present invention described above, the transplantation includes but is not limited to, cell transplantation, organ transplantation or tissue transplantation; wherein cell transplantation includes but is not limited to, stem cell transplantation, regulatory cell transplantation, islet cell transplantation, effector cell transplantation; organ transplantation includes, but is not limited to, renal transplantation, liver transplantation, heart transplantation, small intestine transplantation, lung transplantation, pancreas transplantation, or a joint organ transplantation and other solid or cavity organ transplantation; tissue transplantation includes, but is not limited to, corneal transplantation, limb transplantation, face transplantation, etc. In one embodiment, the transplantation preferably includes renal transplantation, liver transplantation, small intestine transplantation, pancreas transplantation, heart transplantation, lung transplantation, corneal transplantation or stem cell transplantation; in one embodiment, the transplantation of the present invention further is organ transplantation.

According to the uses of the present invention described above, regulatory cells include, but are not limited to, CD4+CD25+ regulatory T cells, CD8+ regulatory T cells, regulatory B cells, regulatory NK cells, NON-T lymphocytes and/or regulatory dendritic cells; wherein as a preferred embodiment, the indicated regulatory cells are NON-T lymphocytes, regulatory dendritic cells; the indicated regulatory dendritic cells include, but are not limited to pDC cells, mDC cells, CD4+mDC cells, CD4-mDC cells, CD8+mDC cells, CD8-mDC cells, or other dendritic cells cultured from monocytes and dendritic cells induced and differentiated from bone marrow cells, etc.

According to the uses of the present invention described above, cinnamon anthranilic acid and its derivatives or a salt thereof include the compound of formula II or a salt thereof:

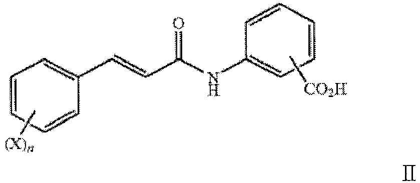

Wherein, X is independent a hydroxyl group, or a halogen, or an alkyl group having 1-4 carbon atoms, or alkoxy having 1-4 carbon atoms, n is an integer of 1, 2 or 3. Further preferably X is methyl, ethyl, propyl, hydroxy, fluoro, chloro, bromo, methoxy group, ethoxy group, propoxy group, a methylene group or an ethylene group. Salts include the inorganic salt. Inorganic acidic salts, for example, include ,but are not limited to hydrochloride, sulphates salts, phosphates, nitrates, carbonates, borates, sulfamate or hydrobromide. Organic salts for example, include, but are not limited to acetate, propionate, butyrate, tartrate, maleate, hydroxy maleate, fumarate, citric acid salts, lactate, mucus, gluconate, benzoate, succinate, oxalate, benzene acetate, methanesulfonate, p-toluenesulfonate, benzenesulfonate, p-aminosalicylic acid salt, aspartic acid salt, glutamic acid salt, edetate, stearates, palmitic , oleate, laurate, pantothenate, tannic acid salt, ascorbic acid salt or valerate. Basic salts include, but are not limited to sodium salts, potassium salts, lithium salts, magnesium salts, calcium salts, ammonium salts or alkyl ammonium salts.
the said cinnamon anthranilic acid and its derivatives or a salt thereof Further preferably is :
2 - [[3 - (2- methyphenyl)-1 - oxo-2 - propenyl] amino] benzoic acid
2 - [[3 - (3- methylphenyl) - 1 - oxo-2 - propenyl] amino] benzoic acid
2 - [[3 - (4-methylphenyl)-1 - oxo-2 - propenyl] amino] benzoic acid
2 - [[3 - (2 - ethylphenyl) - 1 - oxo-2 - propenyl] amino] benzoic acid
2 - [[3 - (3 -ethylphenyl)-1 - oxo-2 - propenyl] amino] benzoic acid
2 - [[3 - (4 - ethylphenyl)-1 - oxo-2 - propenyl] amino] benzoic acid
2 - [[3 - (2 -propylphenyl)-1 - oxo-2 - propenyl] amino] benzoic acid
2 - [[3 - (3 -propylphenyl)-1 - oxo-2 - propenyl] amino] benzoic acid
2 - [[3 - (4 - propylphenyl)-1 - oxo-2- - propenyl] amino] benzoic acid
2 - [[3 - (2-hydroxyphenyl)-l - oxo-2 - propenyl] amino] benzoic acid
2 - [[3 - (3 - hydroxyphenyl) - 1 - oxo-2 - propenyl] amino] benzoic acid
2 - [[3 - (4- hydroxyphenyl)-1-oxo -2 - propenyl] amino] benzoic acid
2 - [[3 - (2-chlorophenyl) - 1 - oxo-2 - propenyl] amino] benzoic acid
2 - [[3 - (3 - chlorophenyl)-1 - oxo - 2-propenyl] amino] benzoic acid
2 - [[3 - (4- chlorophenyl)-1 - oxo - 2 - propenyl] amino] benzoic acid
2 - [[3 - (2 - fluorophenyl)-oxo - 2 - propenyl] amino] benzoic acid
2 - [[3 - (3- fluorophenyl)-1 - oxo - 2 - propenyl] amino] benzoic acid
2 - [[3 - (4 - fluorophenyl)-1 - oxo - 2 - propenyl] amino] benzoic acid
2 - [[3 - (2-bromopheny)l-1 - oxo-2-propenyl] amino] benzoic acid
2 - [[3 - (3-bromophenyl)-oxo -2 - propenyl] amino] benzoic acid
2 - [[3 - (4- bromophenyl)-1 - oxo - 2 - propenyl] amino] benzoic acid
2 - [[3 - (2,3- dimethoxyphenyl)-1 - oxo-2-propenyl] amino] benzoic acid
2 - [[3 - (3,4 - dimethoxyphenyl) -1 - oxo-2 - propenyl] amino] benzoic acid
2 - [[3 - (2,4 - dimethoxyphenyl) - 1 - oxo - 2 -propenyl] amino] benzoic acid
2 - [[3 - (2,3 - dimethylphenyl) -1 - oxo-2-propenyl] amino] benzoic acid
2 - [[3 - (3,4 -dimethylphenyl)-1 - oxo-2 - propenyl] amino] benzoic acid
2 - [[3 - (2,4-dimethylphenyl)-1 - oxo-2-propenyl] amino] benzoic acid
2 - [[3 - (2, 3-diethoxypheny]l-1 - oxo-2 - propenyl] amino] benzoic acid;
2 - [[3 - (3,4 - diethoxypheny]l-1 - oxo-2 - propenyl] amino] benzoic acid;
2 - [[3 - (2,4 - diethoxypheny]l-1 - oxo-2 - propenyl] amino] benzoic acid;
2 - [[3 - (2,3 - diethylpheny]l-1 - oxo-2 - propenyl] amino] benzoic acid;
2 - [[3 - (3,4 - diethylpheny]l-1 - oxo-2 - propenyl] amino] benzoic acid;
2 - [[3 - (2,4 - diethylpheny]l-1 - oxo-2 - propenyl] amino] benzoic acid;
2 - [[3 - (2,3 - dipropylpheny]l-1 - oxo-2 - propenyl] amino] benzoic acid;
2 - [[3 - (3,4 - dipropylpheny]l-1 - oxo-2 - propenyl] amino] benzoic acid;
2 - [[3 - (2,4 - dipropylpheny]l-1 - oxo-2 - propenyl] amino] benzoic acid;
2 - [[3 - (2-methoxyl-3-methyl)pheny]l-1 - oxo-2 - propenyl] amino] benzoic acid;
2 - [[3 - (2-methoxyl-4-methyl)pheny]l-1 - oxo-2 - propenyl] amino] benzoic acid;
2 - [[3 - (3-methoxyl-4- methyl)pheny]l-1 - oxo-2 - propenyl] amino] benzoic acid;
2 - [[3 - (2-methoxyl-3-chloro)pheny]l-1 - oxo-2 - propenyl] amino] benzoic acid;
2 - [[3 - (2-methoxyl-4-chloro)pheny]l-1 - oxo-2 - propenyl] amino] benzoic acid;
2 - [[3 - (3-methoxyl-4-chloro)pheny]l-1 - oxo-2 - propenyl] amino] benzoic acid;
2 - [[3 - (2-methoxyl-3-hydroxy)pheny]l-1 - oxo-2 - propenyl] amino] benzoic acid;
2 - [[3 - (2-methoxyl-4-hydroxy)pheny]l-1 - oxo-2 - propenyl] amino] benzoic acid;
2 - [[3 - (3-methoxyl-4-hydroxy)pheny]l-1 - oxo-2 - propenyl] amino] benzoic acid;
2 - [[3 - (3,4-dimethylene) pheny]l-1 - oxo-2 - propenyl] amino] benzoic acid;
2 - [[3 - (2,3-dimethylene) pheny]l-1 - oxo-2 - propenyl] amino] benzoic acid;
2 - [[3 - (2,3-dimethylene-di-oxo) pheny]l-1 - oxo-2 - propenyl] amino] benzoic acid;
2 - [[3 - (2,3-diethylene-di-oxo) pheny]l-1 - oxo-2 - propenyl] amino] benzoic acid;

The said cinnamon anthranilic acid and its derivatives or a salt thereof Further preferably is 2-[[3 - (3,4 - dimethoxyphenyl) -1 - oxo-2 - propenyl] amino] benzoic acid; other embodiments further include, but are not limited to 3-Hydroxy-anthranilic acid (3-HAA), Picolinic acid (PA), Quinolinic acid (QA), or 3- hydoxykynurenic acid (3-HKA) etc..

According to the uses of the present invention described above, the therapeutically effective amount of indicated cinnamon anthranilic acid and its derivatives or a salt thereof depends on individual differences, the difference depends on the types of species such as human or animals, or the selectivity of different active ingredients, and its range is from 0.01 mg to 500 mg per kilogram of body weight. In large animals, including but not limited to human beings, use dose ranges from 0.5 mg to 5000 mg per day, which depends on the needs can be divided into but not limited to 2 to 4 times. The dose range can be adjusted according to the response to the treatment. Suitable oral dose of cinnamon anthranilic acid is from 1 mg to 5000 mg per day, in general, oral dose of cinnamon anthranilic acid is 100-1000 mg, and the usual oral dose of cinnamon anthranilic acid is 300-600 mg per day. For different recipients, the dose can be appropriately adjusted by the physician according to the body weight, age, sex, degree of illness and other factors of a specific recipient, after oral administration, the drug is rapidly absorbed in the gastrointestinal tract and reaches the peak plasma concentration in 2-3h and is widely distributed to all organs and tissues, wherein bronchial and lung have the highest concentration, liver and kidney and small intestine are the second, the plasma half-life is 5-8.6h, at 24h the blood concentration drops significantly, and at 48h it is difficult to be detected. Parenteral administration dose is 0.01 mg to 300 mg per day. The drug is metabolized in liver, and is mainly discharged via urine.

Compounds having a similar structure with said cinnamon anthranilic acid, and pharmaceutically acceptable salts thereof are known, and they can be prepared using conventional preparation methods in the art. ((see patent US3,940,422, Japan patent Sho-56-40710, Sho-57-36905, Sho-58-17186, Sho-58-48545, Sho-58-55138, Sho-58-55139, Hei-1-28013, Hei-1-50219 and Hei-3-37539). For example yeast fermentation by Saccharomyces Cerevisiae (see 2010 Appl Microbiol Biotechnol. DOI 10.1007/s00253-010-2939-y).

According to uses of the present invention described above, in one embodiment, a composition comprising cinnamon anthranilic acid and its derivatives or a salt thereof comprises that a therapeutically effective amount of one or more types of cinnamon anthranilic acid and its derivatives or a salt thereof and one or more selected from cyclosporine/FK506, Mycophenolate mofetil (MMF), rapamycin, corticosteroids and various types of anti-lymphocyte antibodies to jointly form a composition, or to be jointly administered; when said composition is administered to a recipient, it can be simultaneously administered to the recipient, or are administered in sequence (e.g. cinnamon anthranilic acid and its derivatives or a salt thereof is first administered, and then another one, or vice versa), or are cross administered.

Means to achieve this administration approach can be manual administration, for example, the components of said composition are made into separated individual unit formulations, and when a recipient uses it, he could simply follow the manual instruction (e.g. including but not limited to, taking at the same time, or taking in a chronological order, etc.) to achieve the administration; or conventional formulation preparation methods in the art can be used to prepare the composition into corresponding formulations to achieve the administration, such as said composition is prepared into a simple mixture formulations, and thus just administering the said mixture formulations to a recipient can achieve simultaneous administration; or the composition is made into a controlled release drug formulations which releases one drug first and then releases another drug to achieve administration in sequence of said composition; or it is made into a controlled release formulation of cross-release to achieve cross-release.

According to the uses of the present invention described above, said cinnamon anthranilic acid and its derivatives or a salt thereof or a composition comprising cinnamon anthranilic acid and and one or more selected from cyclosporine/FK506, MMF, rapamycin, corticosteroids and various types of anti-lymphocyte antibodies can be made into different type of pharmaceutical dosage forms with different pharmaceutical excipients using conventional preparation methods in the field, for example, with excipients, disintegrants, binders, lubricants , diluents, buffers, isotonic stabilizing agents, preservatives, humectants, emulsifiers, dispersants, stabilizers and/or dissolving adjuvants, it can be made into oral preparations including but not limited to syrups, granules like fine granules, tablets including but not limited to for example conventional tablets, sustained release tablets, or controlled-release tablets, capsules, such as soft gelatin capsules or hard gelatin capsules; injections including but not limited to for example injection solution, powder for injection, lyophilized preparation for injection, microspheres for injection and/or liposomes for injection, etc.

According to the uses of the present invention described above, the formulation is prepared using conventional preparation methods in the art, for example, tablets are made by mixing mentioned drugs with appropriate excipients, disintegrants, binders, lubricants and other necessary additives, and then press-molding the mixture by a conventional process. If desired, tablets may be covered with a layer of film coat, such as sugar coated tablets and entericcoated tablets. Capsules are made by mixing mentioned drugs with excipients, lubricants and other necessary additives to form various particles or powder, and adding them into a capsule package.

The present invention is verified by experiments that cinnamon anthranilic acid and its derivatives or a salt thereof, or a composition comprising the above can be used in the following treatments:
1) to prevent or treatment the acute rejection in organ, tissue or cell transplant patients in the body of recipients, to promote the generation of regulatory dendritic cells in vivo;
2) to induction of immune tolerance state in organ, tissue or cell transplant patients in the body of recipients, to promote the generation of regulatory dendritic cells in vivo;
3) treatments working on donor recipient cells which are cocultured in vitro, to induce the generation of regulatory cells, and thus to induce immune tolerance in organ, tissue or cell transplant recipients by adoptive transfer of regulatory cells, or to treat patients with autoimmune diseases through adoptive transfer of regulatory dendritic cells.

Experiments show that cinnamon anthranilic acid can inhibit acute rejection of heart graft in rats, significantly prolong the survival term of the transplanted heart. The experiment results have been confirmed in different strains of rats. The mechanism of rat cardiac allograft survival extended by cinnamon anthranilic acid is based on two aspects of regulations, first, the drug can directly kill or inhibit activated lymphocytes, to play the function of the drug against acute rejection, and second, the drug can induce the generation of regulatory cells and suppress the activation of effector cells via regulatory cells, to protect the graft.

In an adoptive transfer experiment of splenocytes from long-term survived rats induced by cinnamon anthranilic acid, in the recipients of next generation, immune tolerance specific to the same donor can be successfully induced, which proves cinnamon anthranilic acid can directly induce the generation of donor specific regulatory cells in vivo, and the cells not only can directly inhibit acute and chronic rejections and extend graft survival, but also can induce the generation of immune tolerance. Thus, we have proved that cinnamon anthranilic acid type drugs can induce the generation of donor specific regulatory cells and therefore induce immune tolerance.

In a further adoptive transfer experiment, the present invention proves that the donor-specific regulatory cells are non-T cell subsets. It is first time that cinnamon anthranilic acid type drugs can induce the generation of non-T regulatory cell subsets is discovered. By flow cytometry sorting different cell subsets for adoptive transfer, the present invention found that cinnamon anthranilic acid type drugs can induce regulatory dendritic cells as well as other non T cells subsets, including plasma cell-derived dendritic cells (pDC) and myeloid-derived dendritic cell subsets (mDC), and so on. This finding reveals one of the cellular mechanism that cinnamon anthranilic acid type drugs induce immune tolerance, and provides a theoretical support for the use of cinnamon anthranilic acid type drugs in the induction of immune tolerance. Dendritic cells are donor antigen-presenting cells, thus the generation of regulatory dendritic cells can be regulated at the source of immune response, which has significant meaning for the successful induction of immune tolerance.

In an in-vitro cell culture experiment, it was found that cinnamon anthranilic acid also can directly kill activated effector cells at a high concentration, and can strongly inhibit the proliferation of effector cells at a low concentration, with a strong anti-acute rejection function. Although the specific mechanism behind this is not clear yet, due to the dual-function mechanism of cinnamon anthranilic acid type drugs - to induce regulatory cells and inhibit or kill effector cells, cinnamon anthranilic acid has a significant role in anti-acute rejection and the induction of immune tolerance after the transplantation.

According to the uses of the present invention, descripted cinnamon anthranilic acid and its derivatives, or a salt thereof can be one of the methodological basis to culture donor-specific regulatory dendritic cells or other non T regulatory cells in vitro, and to induce immune tolerance by adoptively transferring the cells. And this adoptive transfer method of in-vitro induced regulatory cells can be one of the treatment approaches for autoimmune tolerance diseases.

The present invention also have used transgenic approach to induce IDO high expression in transplanted rat heart test group, via adoptive transfer of splenocytes, and it also can induce recipients of the next generation to obtain donor-specific immune tolerance. By continuing adoptive transfer of cell subsets, we have found that it can induce the generation of regulatory dendritic cells, and obtained conclusions similar to the above experiments. Therefore, a metabolite of tryptophan degradation pathway and its derived analogs having biological activities can become a basic drug to induce regulatory dendritic cells, and based on this, it becomes a basic program of inducing immune tolerance by direct in vivo drug administration or adoptive transfer.

Compared with conventional immunosuppressive drugs, the advantages of cinnamon anthranilic acid type drugs of the present invention are not only to promote the generation of regulatory cells, but also to inhibit the proliferation of effector cells and kill activated effector cells. Thus the drugs have dual functions of immune tolerance induction and anti-acute rejection. Further, the mechanism of the drugs mainly acts on the immune lymphocytes of a recipient, without depending on the type of a transplant donor, so the results of this experiment can also be applied to other organ, tissue and cell transplantations, such as renal transplantation, liver transplantation, small intestine transplantation, pancreas transplantation, heart transplantation, lung transplantation, corneal transplantation, stem cell transplantation, etc.

Clinical observations of using cinnamon anthranilic acid type drugs in the treatment of asthma and allergic rhinitis clinically confirm that cinnamon anthranilic acid type drugs have very low toxic side effects, only rarely cause gastrointestinal symptoms such as lack of appetite, nausea and occasionally cause neurological symptoms such as headache, drowsiness, dizziness, etc. When proper measures are taken such as reduction, withdrawal, etc., one can return to normal. Compared with other immunosuppressive agents, when cinnamon anthranilic acid type drugs of the present invention are applied to a transplant recipient, there are no side effects threatening long-term survival of transplant patients and grafts such as renal toxicity, hypertension, diabetes, etc.

### BRIEF DESCRIPTION OF THE DRAWINGS

Figure 1: a curve with circles represents cinnamon anthranilic acid oral experiment group, n=24 ***; a curve with squares represents DMSO oral control group, n=10; log-rank test P<0.0001.
Figure 2: a curve with circles represents cinnamon anthranilic acid oral experiment group, n=6 ***; a curve with squares represents DMSO oral control group, n=6; log-rank test P<0.0007.
Figure 3: a curve with equilateral triangles represents the first generation of spleen cells adoptive transfer group, n=10***; a curve with squares represents the second generation of spleen cells adoptive transfer group, n=8***; a curve with circulars represents cinnamon anthranilic acid treatment group, n=24; log-rank test P<0.0007.
Figure 4: a curve with rhombuses represents adoptive transfer NON-T lymphocytes group n=10***; a curve with squares represents adoptive transfer T lymphocytes group n=5; log-rank test P<0.0001.
Figure 5: a curve with circles represents adoptive transfer mDC cell rich subsets group n=6***; a curve with squares represents adoptive transfer pDC cell subsets group, n=6***; a curve with equilateral triangles represents adoptive transfer T and B cell rich subsets group n=6, log-rank test P<0.0001.
Figure 6: a curve with circles represents adoptive transfer pDC cell rich subsets group n=10; a curve with squares represents adoptive transfer CD4-mDC cell subsets group, n=6; a curve with equilateral triangles represents adoptive transfer CD4+mDC cell subsets group n=6, log-rank test P=0.4134.
Figure 7: Effects of different concentrations of cinnamon anthranilic acid in mixed lymphocyte cultivation on effector cells; wherein, SSC represents cell granule, FSC represents cell size, CD4 and TCR are surface markers of effector cells, and CFSE is a green bioluminescence dye.

### DESCRIPTION OF EMBODIMENTS

The present invention is further illustrated by the following embodiments, but not limited thereto.

### Example 1: Immune tolerance test of heart transplant rats induced by cinnamon anthranilic acid

### 1.1 Experiment Materials

### (1) Drug and Preparation

Cinnamon anthranilic acid: powder (99% purity) was purchased from Xinchen Chemical Co., Ltd. in Shanghai, China. Cinnamon anthranilic acid was dissolved in dark in dimethyl sulfoxide (DMSO, Sigma) and diluted with olive oil to a final concentration of 300mg/ml. The control group was orally treated with a DMSO and olive oil mixture of the same concentration.

### (2) Experiment Animals

Adult Lewis rats LEW-1A, LEW-1W, male, weight of 200-250g, rat age of 6-8 weeks; Brown-Norway (BN) rats, male, weight of 200-250g, rat age of 6-8 weeks; both were purchased from Centre d'Elevage Janvier Animal Centre in France.

### (3) Establishment of Rat Heart Transplantation Model

**a) Heart transplantation animal model with LEW-1W rats as donor, LEW-1A as recipient:** LEW-1W rats were used as donors; donors hearts after being isolated were preserved in saline at 0-4 °C. And male LEW-1A rats were used as recipients, abdominal aorta and inferior vena cava thereof were isolated, and the donors hearts were transplanted to LEW-1A rats abdomens. The donor's aorta and the recipient's abdominal aorta were sutured up with 8-0 Prolene vascular threads, and the donor's pulmonary artery and the recipient's vena cava were sutured up with 8-0 Prolene vascular threads. When blood vessels were opened, the rat's heart would immediately resume beating, (please see Guillonneau C, Hill M, Hubert FX, Chiffoleau E, Hervé C, Xian-Liang Li, et al. CD40lg treatment results in allograft acceptence mediated by CD8+CD45RClow T cells, IFN-γ and indoleamine 2,3 dioxygenase. Journal Clinical Investagtion. 2007 ;117(4):1096-1106 ).
**b) Heart transplantation animal model with BN rats as donor and LEW-1A rats as recipient: BN** rats were used as donors; donors hearts after being isolated were preserved in saline at 0-4 °C. And male LEW-1A rats were used as recipients, abdominal aorta and inferior vena cava thereof were isolated and the donors hearts were transplanted to LEW-1A rats abdomens. The donor's aorta and the recipient's abdominal aorta were sutured up with 8-0 Prolene vascular threads, and the donor's pulmonary artery and the recipient's vena cava were sutured up with 8-0Prolene vascular threads. When blood vessels were opened, the rat's heart would immediately resume beating.

Observation parameters: after the abdomen was closed, palpation was used to check the beating of a transplanted rat heart, to determine graft survival period. That transplanted heart stops beating within 3 weeks was considered as having acute rejection, that transplanted heart stops beating after one month was considered as having chronic rejection, and that transplanted heart beats normally for more than three months was considered as having immune tolerance.

### (4) Statistical Method

The survival statistics of all experimental animals, and the control group were compared and analyzed using a log-rank test method (Log-Rank or Mantel-Cox test). If P<0.05, a result has statistical meaning.

### 1.2. Experiment methods and results

### 1.2.1 Protective effect of cinnamon anthranilic acid on a transplanted rat heart

Experiment Method: 34 recipients from the experiment model established by method a) LEW-1W rats as donors and LEW-1A rats as recipients were divided into study and control groups, wherein the study group had 24 rats, and the control group had 10 rats. Rats in the study group were orally administered cinnamon anthranilic acid (500mg/kg/day) through gastric tube since the day of surgery, twice a day, for four consecutive weeks; rats in the control group were administered the same dose of a DMSO and olive oil mixture. After four weeks, regardless whether a recipient had heart rejection or not, the administration was stopped for observation.

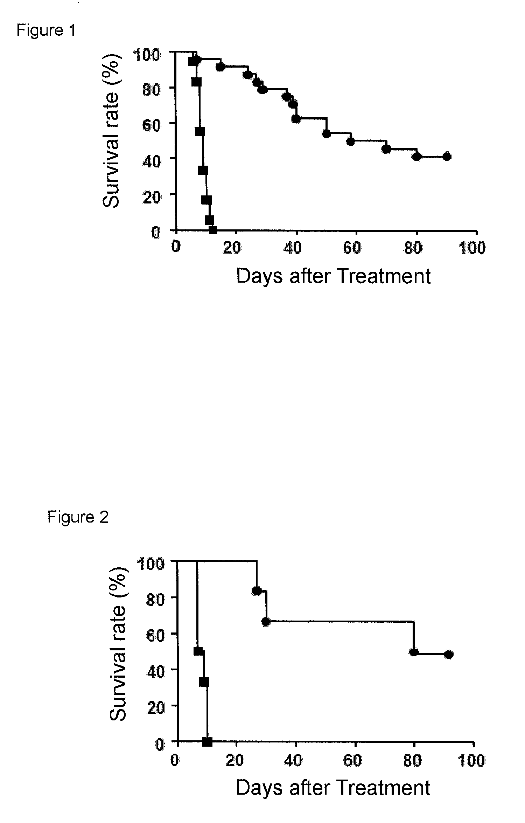

Experiment Results: transplanted hearts of LEW-1A rats in the control group all had acute rejection within 7-12 days, the hearts stopped beating, and the grafts lost their function (n=10). In the group with oral administration of cinnamon anthranilic acid, 92% (2/24) of the recipients overcame acute rejection, and 42% (10/24) of the recipients finally had long-term survival with a state of induced immune tolerance (n=24, P<0.001 relative to the control group). For details, see Table 1 and Figure 1.

**Table 1: Influence of the survival times of LEW-1W donor transplanted rat hearts via Orally taking cinnamon anthranilic acid**

| Orally taking cinnamon anthranilic acid group | | Survival time (day) | DMSO control group | Survival time (day) |
|---|---|---|---|---|
| Recipient 1 | | 80 | recipient1 | 7 |
| Recipient 2 | | 27 | recipient 2 | 8 |
| Recipient 3 | | 15 | recipient 3 | 9 |
| recipient 4 | | 90 | recipient 4 | 10 |
| recipient 5 | | 70 | recipient 5 | 10 |
| recipient 6 | | 90 | recipient 6 | 7 |
| recipient 7 | | 90 | recipient 7 | 12 |
| recipient 8 | | 90 | recipient 8 | 11 |
| recipient 9 | | 90 | recipient 9 | 11 |
| recipient 10 | | 90 | recipient 10 | 8 |
| recipient 11 | | 90 | | |
| recipient 12 | | 40 | | |
| recipient 13 | | 37 | | |
| recipient 14 | | 24 | | |
| recipient 15 | | 29 | | |
| recipient 16 | | 39 | | |
| recipient 17 | | 7 | | |
| recipient 18 | | 58 | | |
| recipient 19 | | 50 | | |
| recipient 20 | | 50 | | |
| recipient 21 | | 40 | | |
| recipient 22 | | 90 | | |
| recipient 23 | | 90 | | |
| recipient 24 | | 90 | | |
| | | | | |

| | log-rank test method | | | |
|---|---|---|---|---|
| | X2 | | 36.82 | |
| | df | | 1 | |
| | P value | | < 0.0001 | |
| | P value analysis | | *** | |
| | Significance of survival curve? | | Yes | |

Conclusion: Orally taking cinnamon anthranilic acid made 92% of the recipients to overcome acute rejection, significantly prolonged the survival term of transplanted rat hearts and induced immune tolerance in 42% of the recipients.

### 1.2.2 Protective effects of cinnamon anthranilic acid on transplanted rat hearts of different rat strains

Experiment Methods: 12 rats from the experiment model established by method b) BN rats as donors and LEW-1A rats as recipients were used and divided into study and control groups, 6 rats for each group. Rats in the study group were orally administered cinnamon anthranilic acid (500mg/kg/day) through gastric tube since the day of surgery, twice a day, for four consecutive weeks; rats in the control group were administered the same dose of a DMSO and olive oil mixture. After four weeks, regardless whether a recipient had heart rejection or not, the administration was stopped for observation.

Experiment Results: As shown in Table 2, transplanted hearts of LEW-1A rats in the control group all had acute rejection within 7-12 days (n=6), and in the group with oral administration of cinnamon anthranilic acid, 100% (6/6) of the recipients overcame acute rejection, and 50% (3/6) of the recipients finally had long-term survival with a state of induced immune tolerance (P<0.001 relative to the control group). For details, see Table 2 and Figure 2.

**Table 2: Influence of the survival times of BN donor transplanted rat hearts via Orally taking cinnamon anthranilic**

| Orally taking cinnamon anthranilic acid group | | Survival time (day) | DMSO control group | | Survival time (day) |
|---|---|---|---|---|---|
| recipient1 | | 80 | recipient1 | | 7 |
| recipient 2 | | 27 | recipient 2 | | 7 |
| recipient 3 | | 30 | recipient 3 | | 10 |
| recipient 4 | | 90 | recipient 4 | | 9 |
| recipient 5 | | 90 | recipient 5 | | 10 |
| recipient 6 | | 90 | recipient 6 | | 7 |
| | | | | | |

| | log-rank test method | | | | |
|---|---|---|---|---|---|
| | X2 | | | 11.62 | |
| | df | | | 1 | |
| | P value | | | 0.0007 | |
| | P value analysis | | | *** | |
| | Significance of survival curve? | | | Yes | |

Conclusion: Orally taking cinnamon anthranilic acid can induce immune tolerance in heart transplantation models with different rat strains.

### Example 2: Immune tolerance test of heart transplant rats induced by cinnamon anthranilic acid via adoptive transfer

### 2.1. Experiment Materials

Cinnamon anthranilic acid: powder (99% purity) was purchased from Xinchen Chemical Co., Ltd. in Shanghai, China. Cinnamon anthranilic acid was dissolved in dark in dimethyl sulfoxide (DMSO, Sigma) and diluted with olive oil to a final concentration of 300mg/ml. The control group was orally treated with a DMSO and olive oil mixture of the same concentration.

### (2) Experiment Animals

Adult Lewis rats LEW-1A, LEW-1W, male, weight of 200-250g, rat age of 6-8 weeks; Brown-Norway (BN) rats, male, weight of 200-250g, rat age of 6-8 weeks; both were purchased from Centre d'Elevage Janvier Animal Centre in France.

### (3) Establishment of rat heart transplantation model

**a) Heart transplantation animal model with LEW-1W rats as donors, LEW-1A as recipients:** LEW-1W rats were used as donors; donors hearts after being isolated were preserved in saline at 0-4 °C. And male LEW-1A rats were used as recipients, abdominal aorta and inferior vena cava thereof were isolated, and the donors hearts were transplanted to LEW-1A rats abdomens. The donor's aorta and the recipient's abdominal aorta were stitched up with 8-0 Prolene vascular threads, and the donor's pulmonary artery and the recipient's vena cava were stitched up with 8-0 Prolene vascular threads. When blood vessels were opened, the rat's heart would immediately resume beating.

Observation parameters: after the abdomen was closed, palpation was used to check the beating of a transplanted rat heart, to determine graft survival period. That transplanted heart stops beating within 3 weeks was considered as having acute rejection, that transplanted heart stops beating after one month was considered as having chronic rejection, and that transplanted heart beats normally for more than three months was considered as having immune tolerance.

### (4) Statistical Method

The survival statistics of all experiment animals, and the control group were compared and analyzed using log-rank test method (Log-Rank or Mantel-Cox test). If P<0.05, a result has statistical meaning.

### 2.2. Experiment methods and results

### 2.2.1 Immune tolerance introduced by adoptive transferred splenocytes proves the generation of regulatory cells

Preparation of the first generation of adoptive spleen cells: according to the method described above in Section 1.2.1 of Example 1, rats from cinnamon anthranilic acid oral administration group (the same transplantation model and route of administration and donor and recipient strains) were used, spleens of rats survived for more than 60 days were taken and splenocytes or splenocytes subsets were collected through sterile separation.(please see Li Xian Liang*, Menoret S, Chabannes D, et al. Mechanism and localization of CD8 regulatory T cells in a heart transplant model of tolerance. Journal of Immunology. 2010; 185:823-833) After filtration, the cells were diluted in PBS balanced salt solution, and diluted to 30×10⁶/ml, at least equivalent to 30×10⁶ splenocytes.

Preparation of the second generation of adoptive splenocytes: recipients' spleens of the first generation of adoptive transfer long-term survived rat model were taken, splenocytes were separated using the same method as one for the first generation of adoptive splenocytes, and were diluted to the same quantity, i.e. diluted to 30×10⁶/ml, at least equivalent to 30×10⁶ splenocytes.

Experiment Method: 42 rats from the heart transplant experiment model established by method a) LEW-1W rats as donors and LEW-1A rats as recipients were used and divided into three groups. Rats in a cinnamon anthranilic acid experiment group (24 rats) were orally administered cinnamon anthranilic acid drug (500mg/kg/day) through gastric tube since the day of surgery, twice a day, for four consecutive weeks; in the first generation adoptive transfer group (10 rats), the first generation adoptive splenocytes at a concentration of 30×10⁶/ml, at least equivalent to 30×10⁶ splenocytes were used for transplantation transfer; in the second generation adoptive transfer group (8 rats), the second generation adoptive splenocytes at a concentration of 30×10⁶/ml, at least equivalent to 30×10⁶ splenocytes were used for transplantation transfer. All adoptive transfer recipient groups took 4.5Gray dose of X-ray body irradiation at the day before surgery, without any other drug treatment except taking transferred cells.

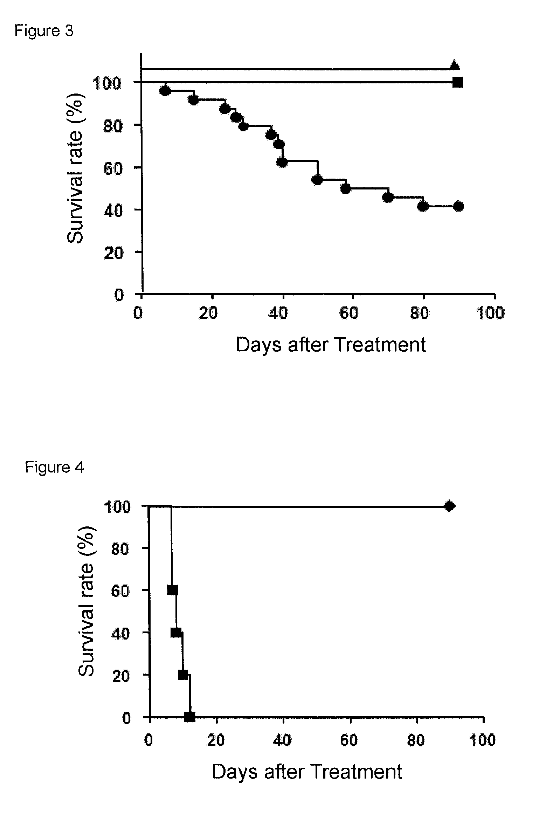

Experiment Results: In the long-term survival cinnamon anthranilic acid oral administration group, spleen cells were used for adoptive transfer, and immune tolerance was successfully induced in all of the first generation recipients (10/10, P<0.001), and immune tolerance was successfully induced in all of the second-generation recipients (8/8, P<0.001) through adoptive transfer of splenocytes collected from the first generation recipients. It proves that regulatory cells generated through cinnamon anthranilic acid oral administration had strong and stable effects, and can induce immune tolerance to specific donors. Moreover, regulatory cells generated via the induction of cinnamon anthranilic acid can mediate the adoptive transfer of immune tolerance. For details, see Table 3 and Figure 3.

**Table 3: The test of Oral admission of cinnamon anthranilic acid can generate regulatory cell-mediated immune tolerance.**

| Oral admission of cinnamon anthranilic acid group | Survival time (day) | First generation of adoptive transfer of splenocytes group | Survival time (day) | Second generation of adoptive transfer of splenocytes group | Survival time (day) |
|---|---|---|---|---|---|
| recipient1 | 80 | recipient1 | 90 | recipient1 | 90 |
| recipient 2 | 27 | recipient 2 | 90 | recipient 2 | 90 |
| recipient 3 | 15 | recipient 3 | 90 | recipient 3 | 90 |
| recipient 4 | 90 | recipient 4 | 90 | recipient 4 | 90 |
| recipient 5 | 70 | recipient 5 | 90 | recipient 5 | 90 |
| recipient 6 | 90 | recipient 6 | 90 | recipient 6 | 90 |
| recipient 7 | 90 | recipient 7 | 90 | recipient 7 | 90 |
| recipient 8 | 90 | recipient 8 | 90 | recipient 8 | 90 |
| recipient 9 | 90 | recipient 9 | 90 | | |
| recipient 10 | 90 | recipient 10 | 90 | | |
| recipient 11 | 90 | | | | |
| recipient 12 | 40 | | | | |
| recipient 13 | 37 | | | | |
| recipient 14 | 24 | | | | |
| recipient 15 | 29 | | | | |
| recipient 16 | 39 | | | | |
| recipient 17 | 7 | | | | |
| recipient 18 | 58 | | | | |
| recipient 19 | 50 | | | | |
| recipient 20 | 50 | | | | |
| recipient 21 | 40 | | | | |
| recipient 22 | 90 | | | | |
| recipient 23 | 90 | | | | |
| recipient 24 | 90 | | | | |
| | | | | | |

| | log-rank test method | | | | |
|---|---|---|---|---|---|
| | X2 | | | 14.66 | |
| | df | | | 2 | |
| | P value | | | 0.0007 | |
| | P value analysis | | | *** | |
| | Significance of survival curve? | | | Yes | |

Conclusion: Oral admission of cinnamon anthranilic acid can generate regulatory cell-mediated immune tolerance.

### 2.2.2 Adoptive transfer test of NON-T cell-mediated immune tolerance

Preparation of T cells and non-T adoptive transfer cells: the first generation of adoptive splenocytes prepared in Section 2.2.1 were used, and the splenocytes were preserved with low temperature (4 °C) mix labeled with TCR or CD45R, 3.2.3, CD12, His24, OX42, CD11c antibodies respectively. After washing away unlabeled antibodies, the splenocytes were purified using immuno-magnetic bead negative selection method, to remove TCR+ or CD45R+, 3.2.3+, CD12+, His24+, OX42+, CD11c+cells (antibody concentration was 5-10µg/ml), and to get NON-T cell rich subsets or T cell rich subsets.

Experiment Method: 15 rats from the heart transplant experiment model established by method a) LEW-1W rats as donors and LEW-1A rats as recipients were used and divided into two groups. One was adoptive transfer NON-T cell group (10 rats), and the other was adoptive transfer T cell group (5 rats). All adoptive transfer recipients took 4.5Gray dose of X-ray body irradiation at the day before surgery, without any other drug treatment except taking transferred cells. Rats in the adoptive transfer T cell group took T cells at least equivalent to 30×10⁶ splenocytes and immediately transferred to the next generation recipients at the same day of the transplantation, and the adoptive transfer NON-T cell group took the same amount of NON-T cell transfer.

Experiment Results: All transplanted hearts in the adoptive transfer NON-T cell group had long-term survival (10/10), but all in the T-cell group had acute rejection (5/5). It proves that immune tolerance is mediated by non-T cells, and T cells in the recipients have no regulatory function of inducing immune tolerance. For details, see Table 4 and Figure 4.

**Table 4: Immune tolerance induced by cinnamon anthranilic acid is mediated by non-T cells.**

| Adoptive transfer NON-T group | | Survival time (day) | Adoptive transfer T cell group | | Survival time (day) |
|---|---|---|---|---|---|
| recipient1 | | 90 | recipient1 | | 10 |
| recipient 2 | | 90 | recipient 2 | | 7 |
| recipient 3 | | 90 | recipient 3 | | 8 |
| recipient 4 | | 90 | recipient 4 | | 7 |
| recipient 5 | | 90 | recipient 5 | | 12 |
| recipient 6 | | 90 | | | |
| recipient 7 | | 90 | | | |
| recipient 8 | | 90 | | | |
| recipient 9 | | 90 | | | |
| recipient 10 | | 90 | | | |
| | | | | | |

| | log-rank test method | | | | |
|---|---|---|---|---|---|
| | X2 | | | 18.21 | |
| | df | | | 1 | |
| | P value | | | < 0.0001 | |
| | P value analysis | | | *** | |
| | Significance of survival curve? | | | Yes | |

Conclusion: immune tolerance induced by cinnamon anthranilic acid is mediated by non-T cells.

### 2.2.3 Adoptive transfer test of dendritic cell-mediated immune tolerance

Preparation of cells of pDC rich, mDC rich and T-cell and B-cell rich cell subsets: adoptive splenocytes of the first generation prepared in Section 2.2.1 were used, and then a suspension of the splenocytes was subjected to liquid gradient centrifugation using Nicodenz cell separation medium to collect cells of an intermediate layer, which was a mDC-rich cell subset. Cells of a lower layer after the centrifugation were suspended in PBS equilibration buffer, and then were subjected to gradient centrifugation using Ficoll cell separation medium to collect cells of an intermediate layer, which was a pDC-rich cell subset. Cells of a lower layer after Ficoll gradient centrifugation were collected which was a T cell and B cell rich subset.

Experiment Method: 18 rats from the heart transplant experiment model established by method a) LEW-1W rats as donors and LEW-1A rats as recipients were used. All adoptive transfer recipients took 4.5Gray dose of X-ray body irradiation at the day before surgery, without any other drug treatment except taking transferred cells. The rats were divided into three groups. 6 rats were in an adoptive transfer pDC rich cell group which accepted the pDC cell subset (30-50*10⁶ cells) all separated from one spleen, 6 rats were in an adoptive transfer mDC rich cell group which accepted the mDC cell subset (5-15*10⁶ cells) all separated from one spleen, and 6 rats were in an adoptive transfer T, B rich cell subset group which accepted the T, B cell subset (50-100 *10⁶ cells) all separated from one spleen.

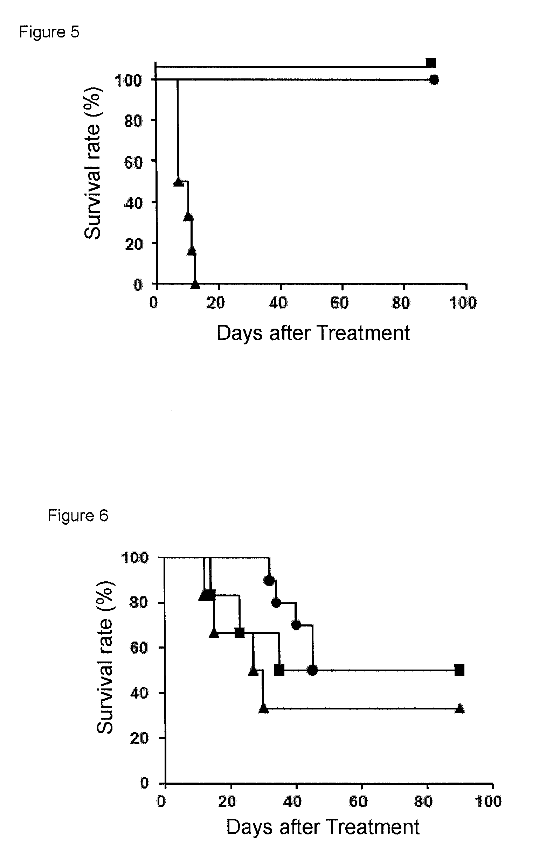

Experiment Results: Transplanted hearts in the adoptive transfer pDC rich cell and mDC rich cell subset groups had long-term survival (6/6), and in the adoptive transfer T, B rich cell subset group acute rejection occurred (6/6). It proves that immune tolerance is cell-mediated by dendritic cells or other NON-T,NON-B subsets, and T, B cells in the recipients do not have regulatory function of inducing immune tolerance. For details, see Table 5 and Figure 5.

**Table 5 : Immune tolerance induced by cinnamon anthranilic acid is mediated by dendritic cells**

| Adoptive transfer enriched mDC group | Survival time (day) | Adoptive transfer enriched pDC group | Survival time (day) | Adoptive transfer enriched T B group | Survival time (day) |
|---|---|---|---|---|---|
| recipient1 | 90 | recipient1 | 90 | recipient1 | 12 |
| recipient 2 | 90 | recipient 2 | 90 | recipient 2 | 11 |
| recipient 3 | 90 | recipient 3 | 90 | recipient 3 | 10 |
| recipient 4 | 90 | recipient 4 | 90 | recipient 4 | 7 |
| recipient 5 | 90 | recipient 5 | 90 | recipient 5 | 7 |
| recipient 6 | 90 | recipient 6 | 90 | recipient 6 | 7 |
| | | | | | |

| | log-rank test method | | | | |
|---|---|---|---|---|---|
| | X2 | | 19.36 | | |
| | df | | 2 | | |
| | P value | | < 0.0001 | | |
| | P value analysis | | *** | | |
| | Significance of survival curve? | | Yes | | |

Conclusion: Immune tolerance induced by cinnamon anthranilic acid is mediated by dendritic cells.

### 2.2.4 Adoptive transfer test of immune tolerance mediated by different types of dendritic cells

Preparation of pDC, CD4+mDC and CD4-mDC adoptive cells: adoptive spleen cells of the first generation prepared in Section 2.2.1 were used. After T lymphocytes (TCR+cells), B cell lymphocytes (CD45R+cells) and NK cells (3.2.3+cells) were removed from the splenocytes using immunomagnetic beads, TCR, CD11c and CD123 were stain marked using fluorescent antibodies (antibody concentration was 5-10µg/ml). Flow cytometry cell sorter was used to collect TCR-CD11c-CD123+cells as pDC, and TCR-CD11c+CD123-cells as mDC.

Experiment Method: Rats from the heart transplant experiment model established by method a) LEW-1W rats as donors and LEW-1A rats as recipients were used. All adoptive transfer recipients took 4.5Gray dose of X-ray body irradiation at the day before surgery, without any other drug treatment except taking transferred cells. The rats were divided into three groups. Rats in an adoptive transfer pDC cell group accepted pDC cell subset (3-5*10⁶ cells) all separated from one spleen, rats in an adoptive transfer CD4-mDC cell group accepted CD4-mDC cell subset (1-3*10⁶ cells) all separated from one spleen, and rats in an adoptive transfer CD4+mDC cell subset group accepted CD4+mDC cell subset (1-3*10⁶ cells) all separated from one spleen.

Experiment Results: 50% of the recipients in the adoptive transfer pDC cell and CD4-mDC cell subset groups had induced immune tolerance (5/10 and 3/6 respectively), and in the CD4+mDC group, 33% of the recipients had induced immune tolerance (2/6). The experiment proves that cinnamon anthranilic acid can induce the generation of different dendritic regulatory cell subsets in donors, and these dendritic regulatory cell subsets can cooperate to mediate immune tolerance together. For details, see Table 6 and Figure 6.

**Table 6 : cinnamon anthranilic acid can induce the generation of different dendritic regulatory cell subsets**

| Adoptive transfer pDCs group | Survival Time (day) | Adoptive transfer CD4-mDCs group | Survival time (day) | | Adoptive transfer CD4+mDCs group | | Survival time (day) |
|---|---|---|---|---|---|---|---|
| recipient1 | 90 | recipient 1 | 23 | | recipient 1 | | 12 |
| recipient 2 | 40 | recipient 2 | 14 | | recipient 2 | | 15 |
| recipient 3 | 34 | recipient 3 | 90 | | recipient 3 | | 90 |
| recipient 4 | 90 | recipient 4 | 90 | | recipient 4 | | 90 |
| recipient 5 | 90 | recipient 5 | 35 | | recipient 5 | | 27 |
| recipient 6 | 45 | recipient 6 | 90 | | recipient 6 | | 30 |
| recipient 7 | 32 | | | | | | |
| recipient 8 | 45 | | | | | | |
| recipient 9 | 90 | | | | | | |
| recipient 10 | 90 | | | | | | |
| | | | | | | | |

| | log-rank test method | | | | | | |
|---|---|---|---|---|---|---|---|
| | X2 | | | 1.767 | | | |
| | df | | | 2 | | | |
| | P value | | | 0.4134 | | | |
| | P value analysis | | | ns | | | |
| | Significance of survival curve? | | | No | | | |

Conclusion: Cinnamon anthranilic acid can induce different dendritic cell subsets to produce regulatory function to mediate immune tolerance.

### Example 3 Effects of mixed lymphocytes culture in cinnamon anthranilic acid of different concentrations on effector cells

Experiment method: From bodies of untreated rats (LEW-1A rats in Example 1), splenocytes were extracted according to the splenocytes extraction method described in Section 2.2.1. Splenocytes were fluorescence-labeled for TCR, CD4 and CD25, and by flow cytometry cell sorter TCR+CD4+CD25-cells were collected as CD4+ effector cells. Splenocytes after the removal of T-lymphocytes (TCR+cells), B cell lymphocytes (CD45R+cells) and NK cells (3.2.3+cells) by immuno-magnetic beads, were marked using fluorescent antibodies for TCR, CD11c and CD123 (antibody concentration was 5-10µg/ml). By flow cytometry cell sorter, TCR-CD11c-CD123+cells were collected as pDC, and TCR-CD11c+CD123-cells were collected as mDC.

Well separated CD4+ effector cells and pDC or mDC and defined regulatory cells, respectively in a proportion of 4/4/1 were added into a 96-well culture plate, 200ul culture medium was added and they were cultured in a 5% CO₂ incubator at 37 degrees Celsius for 4 days. During the cultivation, cinnamon anthranilic acid (1.2-150µmol) or control DMSO solvent was added. Cells were stain labeled after the four days, and the proliferation of effector cells was analyzed by a flow cytometer.

CD4+ effector cells before the mixed culture were marked by living organism dye CFSE (10*6 cells/ml in 5 µM CFSE). Labeled CD4+ effector cells were added to a mixed lymphocyte cultivation test, were marked again using fluorescent antibodies (TCR, CD4) after four days, and then were analyzed by a flow cytometer (TCR+CD4+gate) for effector cell proliferation.

Cinnamon anthranilic acid was added in different concentrations (150µM, 75µM, 37.5µM, 18.7µM, 9.35µM, 4.7µM, 2.35µM, 1.2µM). Cell stain labeling was conducted after 4 days of the culture, and the proliferation of effector cells was analyzed by a flow cytometer.

### Experiment results

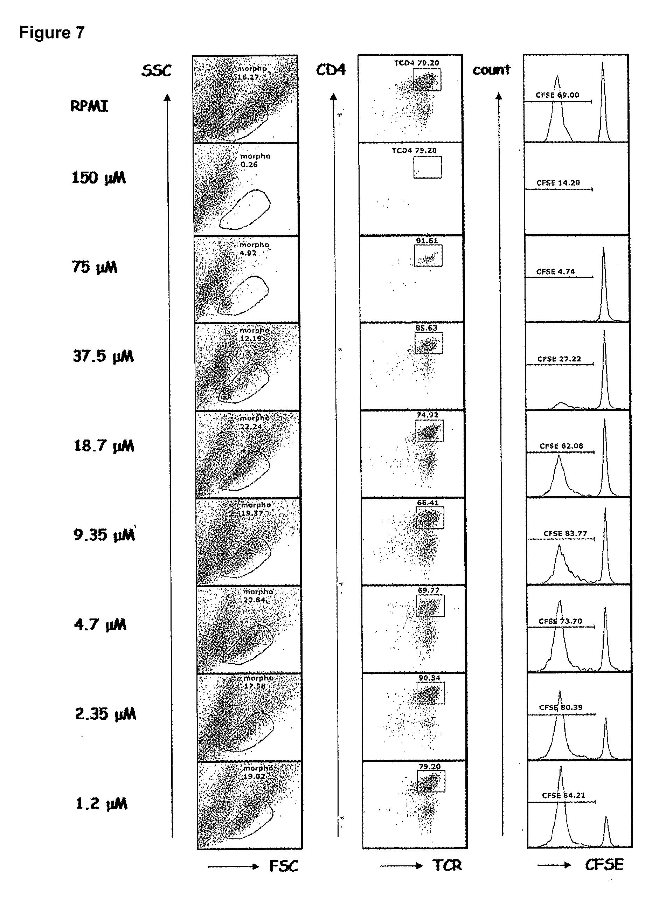

The proliferation of cells in cinnamon anthranilic acid groups is shown in Figure 7: the first column of scatter plot of the flow cytometer shows that, at high concentrations (150µM and 75 µM), cinnamon anthranilic acid can directly kill activated CD4 effector cells, and the amount of cells of the live cell gate and drug concentration have a direct dependent relationship. The second column based on the live cell gate, further sorts out CD4 effector T cells. As observed at the high concentrations, CD4 effector cells were directly killed. In a concentration range of 37.5µM to 9.35µM, on the basis of CD4 effector T cell gate, in the flow cytometry histogram, the cell proliferation analyzed by the CFSE stain technology can be observed in the third column. Under different concentrations, the percentage of division peaks and the value of non-division peaks of CD4 effector T cells, both significantly indicated a dose-dependent inhibitory effect. Thus, cinnamon anthranilic acid can strongly inhibit the proliferation of CD4 effector cells. But at a low concentration range (below 4.7µM), the drug did not show the inhibitory effect.

Above drug dose tests explain the mechanism of the dual function of this medicine. Besides inducing regulatory cells, it can also directly act on effector cells, to kill and inhibit the proliferation and activation of the effector cells. It has dual immunomodulatory capacities.
( The above methods used in the experiments for cell isolation and FACS please see the reference, Ménoret S, Guillonneau C, Bezié S, Caron L, Anegon I, Li Xian-Liang. Phenotypic and functional characterization of CD8(+) T regulatory cells. Methods Mol Biol. 2011;677:63-83 ; Li Xian Liang, Menoret S, Chabannes D, et al. Mechanism and localization of CD8 regulatory T cells in a heart transplant model of tolerance. Journal of Immunology. 2010; 185:823-833)

## Claims

1. A use of cinnamon anthranilic acid and its derivatives, or a salt thereof, or a composition comprising the above in the preparation of a drug for the induction of immune tolerance or for anti-acute rejection after transplantation.

2. The use according to claim 1, wherein the use of said cinnamon anthranilic acid and its derivatives, or a salt thereof, or a composition comprising the above is in the preparation of a drug for inducing a transplant recipient to generate regulatory cells.

3. The use according to claim 2, wherein said regulator cells include CD4+CD25+regulatory T cells, CD8+regulatory T cells, regulatory B cells, NON-T lymphocytes, regulatory NK cells and/or regulatory dendritic cells; Said regulatory cells is NON-T lymphocytes or regulatory dendritic cells; said regulatory dendritic cells include pDC cells, mDC cells, CD4+mDC cells, CD4-mDC cells, CD8+mDC cells, CD8-mDC cells, or dendritic cells cultured from monocytes or dendritic cells induced and differentiated from bone marrow cells.

4. The use according to claim 1, wherein a therapeutically effective amount of cinnamon anthranilic acid and its derivatives, or a salt thereof, or a composition comprising the above is directly administered to a recipient.

5. The use according to claim 1, wherein first a therapeutically effective amount of cinnamon anthranilic acid and its derivatives or a salt thereof, or a composition comprising the above is used in mix cultured donor and recipient cells to induce the generation of regulatory cells, then a therapeutically effective concentration of the regulatory cells are administered to the recipient.

6. The use according to claim 1, wherein said therapeutically effective amount is 1 mg to 5000 mg per day for oral dose, preferably 100-1000 mg per day, 300-600 mg per day for usual oral dose; and 0.01 mg to 300 mg/day for parenteral injection dose.

7. The use according to claim 1, wherein said transplantation includes cell transplantation, organ transplantation or tissue transplantation; wherein the cell transplantation includes stem cell transplantation, regulatory cell transplantation, islet cell transplantation, or effector cell transplantation, etc.; the organ transplantation includes renal transplantation, liver transplantation, heart transplantation, small intestine transplantation, lung transplantation, pancreas transplantation, or a joint organ transplantation; and the tissue transplantation includes corneal transplantation, limb transplantation, or face transplantation, etc.

8. The use according to claim 7, wherein said transplantation includes renal transplantation, liver transplantation, small intestinal transplantation, pancreas transplantation, heart transplantation, lung transplantation, corneal transplantation or stem cell transplantation, pancreatic islet cell transplantation, etc.

9. The use according to any one of the preceding claims, wherein said cinnamon anthranilic acid and its derivatives or a salt thereof includes a compound of the following chemical structure formula II or a salt thereof: 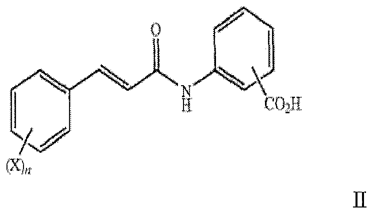 Wherein X is independent a hydroxyl group, or a halogen, or an alkyl group having 1-4 carbon atoms, or alkoxy having 1-4 carbon atoms, n is an integer of 1, 2 or 3, further preferably X is methyl, ethyl, propyl, hydroxy, fluoro, chloro, bromo, methoxy group, ethoxy group, propoxy group, a methylene group or an ethylene group; wherein salts include acidic or basic inorganic salt, said acidic inorganic salts include hydrochloride, sulphates salts, phosphates, nitrates, carbonates, borates, sulfamate or hydrobromide; said basic salts include sodium salts, potassium salts, lithium salts, magnesium salts, calcium salts, ammonium salts or alkyl ammonium salts; or organic salts include acetate, propionate, butyrate, tartrate, maleate, hydroxy maleate, fumarate, citric acid salts, lactate, mucus, gluconate, benzoate, succinate, oxalate, benzene acetate, methanesulfonate, p-toluenesulfonate, benzenesulfonate, p-aminosalicylic acid salt, aspartic acid salt, glutamic acid salt, edetate, stearates, palmitic , oleate, laurate, pantothenate, tannic acid salt, ascorbic acid salt or valerate.

10. The use according to claim 9, wherein said cinnamon anthranilic acid and its derivatives or a salt thereof is:
2 - [[3 - (2 - methyphenyl)-1 - oxo-2 - propenyl] amino] benzoic acid;
2 - [[3 - (3 - methylphenyl) - 1 - oxo-2 - propenyl] amino] benzoic acid;
2 - [[3 - (4 - methylphenyl)-1 - oxo-2 - propenyl] amino] benzoic acid;
2 - [[3 - (2 - ethylphenyl) - 1 - oxo-2 - propenyl] amino] benzoic acid;
2 - [[3 - (3 - ethylphenyl)-1 - oxo-2 - propenyl] amino] benzoic acid;
2 - [[3 - (4 - ethylphenyl)-1 - oxo-2 - propenyl] amino] benzoic acid;
2 - [[3 - (2 - propylphenyl)-1 - oxo-2 - propenyl] amino] benzoic acid;
2 - [[3 - (3 - propylphenyl)-1 - oxo-2 - propenyl] amino] benzoic acid;
2 - [[3 - (4 - propylphenyl)-1 - oxo-2- - propenyl] amino] benzoic acid;
2 - [[3 - (2 - hydroxyphenyl)-1 - oxo-2 - propenyl] amino] benzoic acid;
2 - [[3 - (3 - hydroxyphenyl) - 1 - oxo-2 - propenyl] amino] benzoic acid;
2 - [[3 - (4 - hydroxyphenyl)-1-oxo -2 - propenyl] amino] benzoic acid;
2 - [[3 - (2 - chlorophenyl) - 1 - oxo-2 - propenyl] amino] benzoic acid;
2 - [[3 - (3 - chlorophenyl)-1 - oxo - 2-propenyl] amino] benzoic acid;
2 - [[3 - (4- chlorophenyl)-1 - oxo - 2 - propenyl] amino] benzoic acid;
2 - [[3 - (2 - fluorophenyl)-oxo - 2 - propenyl] amino] benzoic acid;
2 - [[3 - (3- fluorophenyl)-1 - oxo - 2 - propenyl] amino] benzoic acid;
2 - [[3 - (4 - fluorophenyl)-1 - oxo - 2 - propenyl] amino] benzoic acid;
2 - [[3 - (2 - bromopheny)I-1 - oxo-2-propenyl] amino] benzoic acid
2 - [[3 - (3 - bromophenyl)-oxo -2 - propenyl] amino] benzoic acid
2 - [[3 - (4- bromophenyl)-1 - oxo - 2 - propenyl] amino] benzoic acid
2 - [[3 - (2,3 - dimethoxyphenyl)-1 - oxo-2-propenyl] amino] benzoic acid
2 - [[3 - (3,4 - dimethoxyphenyl) -1 - oxo-2 - propenyl] amino] benzoic acid
2 - [[3 - (2,4 - dimethoxyphenyl) - 1 - oxo - 2 -propenyl] amino] benzoic acid
2 - [[3 - (2,3 - dimethylphenyl) -1 - oxo-2-propenyl] amino] benzoic acid
2 - [[3 - (3,4 - dimethylphenyl)-1 - oxo-2 - propenyl] amino] benzoic acid
2 - [[3 - (2,4 - dimethylphenyl)-1 - oxo-2-propenyl] amino] benzoic acid
2 - [[3 - (2, 3 - diethoxypheny]l-1 - oxo-2 - propenyl] amino] benzoic acid;
2 - [[3 - (3,4 - diethoxypheny]l-1 - oxo-2 - propenyl] amino] benzoic acid;
2 - [[3 - (2,4 - diethoxypheny]l-1 - oxo-2 - propenyl] amino] benzoic acid;
2 - [[3 - (2,3 - diethylpheny]l-1 - oxo-2 - propenyl] amino] benzoic acid;
2 - [[3 - (3,4 - diethylpheny]l-1 - oxo-2 - propenyl] amino] benzoic acid;
2 - [[3 - (2,4 - diethylpheny]l-1 - oxo-2 - propenyl] amino] benzoic acid;
2 - [[3 - (2,3 - dipropylpheny]l-1 - oxo-2 - propenyl] amino] benzoic acid;
2 - [[3 - (3,4 - dipropylpheny]l-1 - oxo-2 - propenyl] amino] benzoic acid;
2 - [[3 - (2,4 - dipropylpheny]l-1 - oxo-2 - propenyl] amino] benzoic acid;
2 - [[3 - (2-methoxyl-3-methyl)pheny]l-1 - oxo-2 - propenyl] amino] benzoic acid;
2 - [[3 - (2-methoxyl-4-methyl)pheny]l-1 - oxo-2 - propenyl] amino] benzoic acid;
2 - [[3 - (3-methoxyl-4-methyl)pheny]l-1 - oxo-2 - propenyl] amino] benzoic acid;
2 - [[3 - (2-methoxyl-3-chloro)pheny]l-1 - oxo-2 - propenyl] amino] benzoic acid;
2 - [[3 - (2-methoxyl-4-chloro)pheny]l-1 - oxo-2 - propenyl] amino] benzoic acid;
2 - [[3 - (3-methoxyl-4-chloro)pheny]l-1 - oxo-2 - propenyl] amino] benzoic acid;
2 - [[3 - (2-methoxyl-3-hydroxy)pheny]l-1 - oxo-2 - propenyl] amino] benzoic acid;
2 - [[3 - (2-methoxyl-4-hydroxy)pheny]l-1 - oxo-2 - propenyl] amino] benzoic acid;
2 - [[3 - (3-methoxyl-4-hydroxy)pheny]l-1 - oxo-2 - propenyl] amino] benzoic acid;
2 - [[3 - (3,4-dimethylene) pheny]l-1 - oxo-2 - propenyl] amino] benzoic acid;
2 - [[3 - (2,3-dimethylene) pheny]l-1 - oxo-2 - propenyl] amino] benzoic acid;
2 - [[3 - (2,3-dimethylene-di-oxo) pheny]l-1 - oxo-2 - propenyl] amino] benzoic acid;or
2 - [[3 - (2,3-diethylene-di-oxo) pheny]l-1 - oxo-2 - propenyl] amino] benzoic acid.

11. The use according to claim 10, wherein said cinnamon anthranilic acid and its derivatives is: 2-[[3-(3,4-dimethoxyl)-phenyl-1-oxo-2-propenyl]amino]benzoic acid.

12. The use according to claim 1, wherein said composition is a combination of cinnamon anthranilic acid and its derivatives or a salt thereof with one or more selected from cyclosporine/FK506, MMF, rapamycin, corticosteroids and anti-lymphocyte antibodies; preferably is a combination of cinnamon anthranilic acid with rapamycin.

13. The use according to any one of the preceding claims, wherein the cinnamon anthranilic acid and its derivatives, or a salt thereof, or a composition comprising the above is in a form of a formulation, and the formulation includes syrups; granules preferably fine granules; tablets preferably conventional tablets, sustained release tablets, or controlled-release tablets; capsules, preferably soft gelatin capsules or hard gelatin capsules; injections, preferably injection solution, powder for injection, lyophilized preparation for injection, microspheres for injection or liposomes for injection.

14. A composition for inducing immune tolerance or anti-acute rejection after transplantation, wherein said composition comprises a combination of cinnamon anthranilic acid and its derivatives or a salt thereof with one or more selected from cyclosporine/FK506, MMF, rapamycin, corticosteroids and anti-lymphocyte antibodies; preferably a combination of cinnamon anthranilic acid with rapamycin.
